# EUROPEAN PATENT APPLICATION

(11) **EP 2 921 463 A1**
(43) Date of publication of application: **23.09.2015**
(21) Application number: 13855669.1
(22) Date of filing: 23.08.2013
(51) Int. Cl.: C03C 17/38, G01N 21/55

(54) **SURFACE PLASMON RESONANCE SENSOR CHIP, AND PREPARATION METHOD AND APPLICATION THEREOF**

(30) Priority: 13.11.2012 CN 201210453443
(71) Applicant: Technical Institute of Physics and Chemistry of the Chinese Academy of Sciences, Haidian District Beijing 100190 (CN)
(72) Inventor: WANG, Pengfei, Beijing 100190 (CN); LAN, Minhuan, Beijing 100190 (CN); ZHANG, Hongyan, Beijing 100190 (CN)
(74) Representative: Serjeants LLP
(86) International application number: PCT/CN2013/082150
(87) International publication number: WO 2014/075482

(57) **Abstract**

Disclosed is a surface plasmon resonance sensor chip, comprising a glass substrate layer, a gold film layer and a probe molecule layer. The gold film layer is disposed on the glass substrate layer and the probe molecule layer is disposed on the gold film layer. Also disclosed is a method for preparing the surface plasmon resonance sensor chip. By means of a surface plasmon resonance spectrum generated by the surface of the gold film disposed on the glass substrate, the content of lipopolysaccharide in an aqueous solution is detected in a fast, simple, quantitative and ultra-sensitive way.

## Description

### FIELD OF THE INVENTION

The present invention relates to the technical field of the preparation of a surface plasmon sensor chip, particularly to a surface plasmon resonance sensor chip for detecting lipopolysaccharide, and preparation method and application thereof.

### BACKGROUND OF THE INVENTION

Lipopolysaccharide (hereinafter called LPS for short) is a polymer, which consists of fats and polysaccharides linked by a covalent bond. LPS is a main component of the outer membrane of gram-negative bacterium and a powerful bacterial toxin which is called endotoxin. LPS is released when gram-negative bacteria such as *E*. *coli* and *Salmonella enterica* multiplies or cracks. Gram-negative bacteria in the human body releases a large number of LPS inside its cell wall, which can cause the body infection or inflammation and further lead to a serious disease which threatens human health-sepsis. Due to the high toxicity of LPS, the content of LPS in purified water or water for injection must be detected to effectively control microbial contamination and control the level of bacterial endotoxin so as to ensure the safety of water quality. Therefore, researchers have been working on inventing a method for detecting LPS in an aqueous solution with high selectivity and ultra-sensitivity. So far, gel method is the most common clinical way for detecting LPS, which can detect LPS qualitatively, semi-quantitatively or highly sensitively. However, this method must use one kind of paleontological (specifically, horseshoe crab) blood, so the long-term and extensive use of this method must be limited. In addition, this method involves complicated steps, is very sensitive to temperature and pH of the environment, and shows positive for some other carbohydrates. Although a photochemical sensor has many advantages such as high selectivity, fast response and convenience in use, its sensitivity is subject to the limitations of fluorescence signal and unable to meet the actual detection requirements of LPS (pM). Therefore, it is imperative to develop a detection method with high sensitivity, high selectivity and low cost for detecting the content of LPS in an aqueous solution.

Surface Plasmon Resonance (hereinafter called SPR for short) refers to a resonance wave which is formed on a metal-dielectric interface and may change light wave transmission, when the light wave impinges on the metal-dielectric interface. That is to say, when an incident light penetrates into a glass prism at a particular angle, a total internal reflection evanescent wave will be generated. The penetration distance of such wave is about 300 nm, causing free electrons on the metal surface to generate a surface plasmon. When the surface plasmon resonance has the same frequency as that of the evanescent wave, a resonance will occur and the incident light is absorbed, resulting in a sharp decline of the reflected light energy, therefore, a resonance peak appears on a reflectance spectroscopy (namely the minimum value of reflection intensity). Any small changes of the refractive index and conformation of a surface medium will result in a shift of the incident angle. Such changes are captured by a detector and converted into corresponding spectra. Surface plasmon wave is very sensitive to small changes of the refractive index and conformation of a medium, therefore, when a sample to be tested is contacted with a metal film in which surface plasma resonance occurs and interacted with each other, the dielectric constant, refractive index and conformation of the film will change, and thus such changes will affect the resonance condition and cause the shift of the resonance peak. The resonance principle of a SPR sensor bring advantages as follows: such sensor, compared with the conventional biological testing means or chemical testing means, can realize real-time, dynamic and particularly ultra-sensitive detection. Recently, the SPR sensor has been widely used in many areas such as environmental health, food safety and disease diagnosis. The SPR sensor is mainly used in the detection of the interaction of biological molecules and other materials and dynamics molecules, etc. But there is no SPR sensor suitable for detecting LPS, not to mention a SPR technology for real-time, fast, simple, quantitative, ultra-sensitive detection of the content of LPS in an aqueous solution, particularly the detection method of the content of LPS in water for injection.

### SUMMARY OF THE INVENTION

The first technical problem to be solved by the present invention is to provide a surface plasmon resonance sensor chip.

The second technical problem to be solved by the present invention is to provide a method for preparing the surface plasmon resonance sensor chip. By means of a surface plasmon resonance spectrum generated by the surface of the gold film disposed on a glass substrate, the content of LPS in an aqueous solution is detected in a fast, simple, quantitative and ultra-sensitive way.

The third technical problem to be solved by the present invention is to provide a use of the surface plasmon resonance sensor chip.

The present invention provides a surface plasmon resonance sensor chip, comprising a glass substrate layer, a gold film layer and a probe molecule layer. The gold film layer is disposed on the glass substrate layer and the probe molecule layer is disposed on the gold film layer. The probe molecule layer is the layer of one or more probe molecules selected from the group consisting of the following structures: 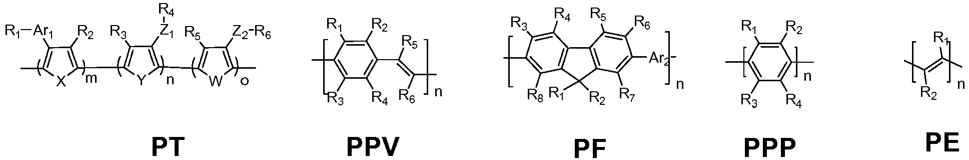 wherein, Ar₁ is thiophene, pyrrole, benzene, naphthalene, anthracene, pyrene, indole, coumarin, fluorescein, carbazole, rhodamine, cyano dyes, fluorene or quinoline;

Ar₂ is one of the following structural formulas: 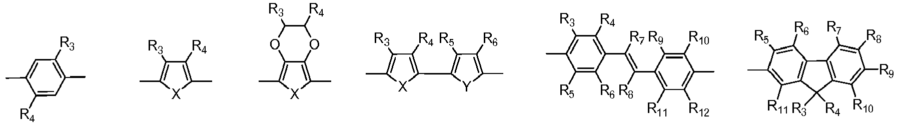 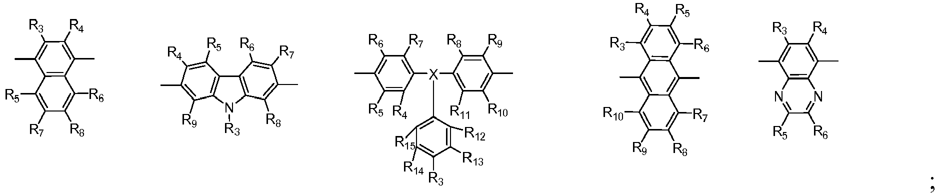 X, Y and W is O, S, N-R₅ or Si-R₆R₇, respectively;
Z₁, Z₂, R₁, R₂, R₃, R₄, R₅, R₆, R₇, R₈, R₉, R₁₀, R₁₁, R₁₂, R₁₃, R₁₄ and R₁₅ are hydrogen atoms; alkyl group, hydroxyl group, mercapto group, carboxyl group, amide, acid anhydride, alkenyl, alkynyl, aryl group, ester group and ether group of 1-18 carbon atoms; amino, cyano group, quaternary ammonium salt, sulphonate, phosphate or polyethylene glycol group, respectively;
m, n and o are natural numbers in the range of 0-10,000, while m, n and o do not represent 0 simultaneously.

In the context, "alkyl group, hydroxyl group, mercapto group, carboxyl group, amide, acid anhydride, alkenyl, alkynyl, aryl group, ester group and ether group of 1-18 carbon atoms" refers to the number of carbon atoms of alkyl group, hydroxyl group, mercapto group, carboxyl group, amide, acid anhydride, alkenyl, alkynyl, aryl group, ester group and ether group is in the range of 1-18.

The thickness of the gold film layer is in the range of 10-60 nm; the thickness of the probe molecule layer is in the range of 1-100 nm; the coverage rate of the probe molecule layer on the gold film layer is in the range of 5%-100%. The coverage rate of the probe molecule layer on the gold film layer can be semi-quantitatively determined by Atomic Force Microscope (AFM). After applied on the gold film layer, the probe molecule will self-assemble to form a granular structure; the surface-modified mercapto group of the probe molecule contacts with the gold film.

All the probe molecules can be purchased commercially or synthesized according to the prior literatures.

The present invention further provides a method for preparing the surface plasmon resonance sensor chip, comprising the following steps:
1) plating gold (Au) on the surface of the glass substrate;
2) soaking the glass substrate obtained from step 1) completely into a probe molecule solution with a concentration of 0.01-1000 mg/mL, for 5 minutes-24 hours;
3) taking the glass substrate out of the probe molecule solution and washing it repeatedly with water to obtain the surface plasmon resonance sensor chip.

The said probe molecule solution is the solution of one or more substances selected from the group consisting of the following structures: 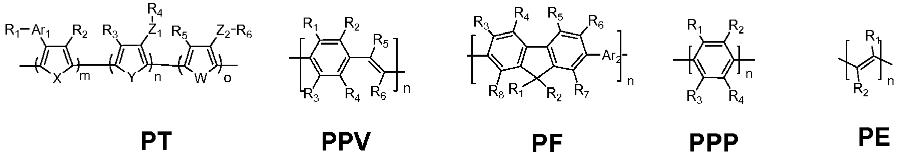 wherein, Ar₁ is thiophene, pyrrole, benzene, naphthalene, anthracene, pyrene, indole, coumarin, fluorescein, carbazole, rhodamine, cyano dyes, fluorene or quinoline;
Ar₂ is one of the following structural formulas: 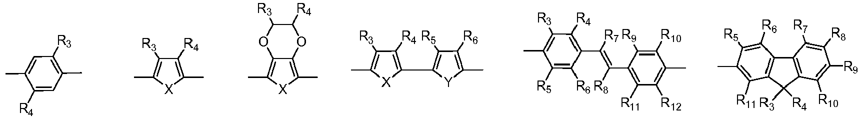 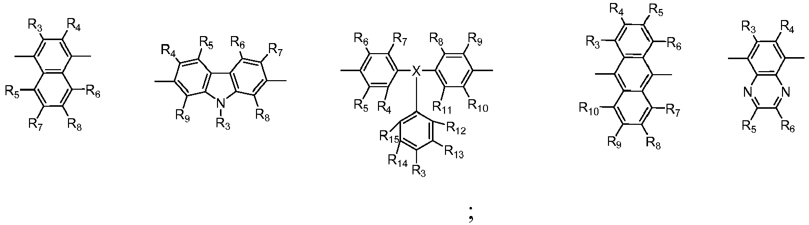 X, Y and W are O, S, N-R₅ or Si-R₆R₇, respectively;
Z₁, Z₂, R₁, R₂, R₃, R₄, R₅, R₆, R₇, R₈, R₉, R₁₀, R₁₁, R₁₂, R₁₃, R₁₄ and R₁₅ are hydrogen atoms; alkyl group, hydroxyl group, mercapto group, carboxyl group, amide, acid anhydride, alkenyl, alkynyl, aryl group, ester group and ether group of 1-18 carbon atoms; amino, cyano group, quaternary ammonium salt, sulphonate, phosphate or polyethylene glycol group, respectively.
m, n and o are natural numbers in the range of 0-10,000, while m, n and o do not represent 0 simultaneously.

The thickness of the gold film is in the range of 10-60 nm. The coverage rate of the probe molecule on the gold film is in the range of 5%-100%.

In the step 3), alternatively, the water may be selected from double distilled water, three times distilled water, four times distilled water, ultra-pure water, etc.

The washed fluid is analyzed with High-performance liquid chromatography (HPLC) after the glass substrate is washed with water for one or more times. The glass substrate can be considered to have been washed clean when the signal of the probe molecule can not be detected. The purpose of washing is to remove the probe molecules adsorbed on the surface of the gold film by physical absorption way.

The concentration and the soaking time of the probe molecule will ultimately affect the coverage rate of the probe molecule on the gold film, and thereby affect the sensitivity of the chip.

Finally the obtained sensor chip is stored in double distilled water for use.

The above reaction can be carried out at room temperature.

The plating step 1) uses vacuum evaporation or magnetron sputtering technique. The vacuum degree of experimental equipment for Au plating is 1 × 10⁻⁴ Pa. The thickness of the gold film can be controlled precisely to be in the range of 10-60 nm by adjusting the frequency change (10-60 Hz) and the evaporation rate of 0.1 Å/s of the film thickness meter.

Further, the solvent of the probe molecule solution is selected from:
physiological saline, HEPES buffer solution or phosphate buffer solution; or
one solvent selected from the group consisting of methanol, ethanol, methyl cyanide, dichloromethane, chloroform, tetrahydrofuran, methyl-sulfoxide, N,N-dimethyl formamide, and N,N-dimethyl acetamide or a mixed solvent thereof; or
a mixture of the above one solvent or mixed solvent and water in any proportions.

The present invention further provides a method for preparing the surface plasmon resonance sensor chip, comprising the following steps:
(1) plating Au on the surface of the glass substrate;
(2) soaking the glass substrate obtained from step (1) into compound S1 solution with a concentration of 0.01-1000 mmol/L, for 1-24 hours; then washing the glass substrate repeatedly with water for use; mixing the probe molecules with NHS, EDC and a solvent at a mass ratio of 1: 0.1-100: 0.1 -100: 1-1000 to obtain a mixed solution; soaking the above glass substrate into the mixed solution for 5 min-24 h, then taking out the glass substrate and washing it with ethanol and water repeatedly;
   wherein, the structural formula of compound S1 is: 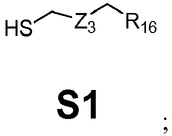 wherein, Z₃ and R₁₆ are hydrogen atoms, respectively; alkyl group, hydroxyl group, mercapto group, carboxyl group, amide, acid anhydride, alkenyl, alkynyl, aryl group, ester group and ether group of 1-18 carbon atoms; amino, cyano group or polyethylene glycol group;
(3) taking out the glass substrate and washing it repeatedly with water to obtain a surface plasmon resonance sensor chip.

The limitation for structure of the probe molecule is the same as that of the above paragraph. NHS is N- hydroxysuccinimide; EDC is 1-ethyl-(3-dimethylaminopropyl) carbodiimide.

The thickness of the gold film is in the range of 10-60 nm. The coverage rate of the probe molecule on the gold film is in the range of 5%-100%.

In step (2), the alternative water is as follows: double distilled water, three times distilled water, four times distilled water, ultra-pure water, etc. The flushing fluid is detected with HPLC, and then the glass substrate can be considered to have been washed clean when the signal of compound S1 can not be detected. Unclean wash may affect the coverage rate of the probe molecule on the gold film. The soaked glass substrate is first washed with ethanol and then with water. Compound S1 can be purchased commercially or synthesized by the existing literature.

The changes such as the ratio and soaking time of the probe molecule and other substance will ultimately affect the coverage rate of the probe molecule on the gold film and thereby affect the sensitivity of the chip.

The above reaction can be carried out at room temperature.

Plating film of step (1) uses vacuum evaporation or magnetron sputtering technique. The vacuum degree of experimental equipment for Au plating is 1 × 10⁻⁴ Pa. The thickness of the gold film can be controlled precisely in the range of 10-60 nm by adjusting the frequency change (10-60 Hz) and the evaporation rate of 0.1 Å/s of the film thickness meter.

Further, the compound S1 dissolves in physiological saline, HEPES buffer solution and phosphate buffer solution; or one solvent selected from the group consisting of methanol, ethanol, methyl cyanide, dichloromethane, chloroform, tetrahydrofuran, methyl-sulfoxide, N,N-dimethyl formamide and N,N-dimethyl acetamide or a mixed solvent thereof; or a mixture of the one solvent or the mixed solvent and water in any proportions, to form compound S1 solution.

Further, the solvent is selected from physiological saline, HEPES buffer solution or phosphate buffer solution; or one solvent selected from the group consisting of methanol, ethanol, methyl cyanide, dichloromethane, chloroform, tetrahydrofuran, methyl-sulfoxide, N,N-dimethyl formamide and N,N-dimethyl acetamide or a mixed solvent thereof; or a mixture of the one or the mixed solvent and water in any proportions.

The present invention further provides a use of the surface plasmon resonance sensor chip for detecting LPS in an aqueous solution.

Further, in the use, the sensor chip is mounted into an angle modulation type or a wavelength modulation type surface plasmon resonance sensor apparatus. LPS aqueous solutions with various concentrations are introduced into a flow cell and LPS is detected by detecting a surface plasmon resonance peak shift.

Further, the amount of the surface plasmon resonance peak shift is linearly proportional to the concentration of the introduced LPS in a corresponding range and such linear relationship can be used to detect LPS quantitatively.

Compared to the prior art, the present invention has the following beneficial effects:
1) The surface plasmon resonance sensor technology for detecting LPS in an aqueous solution is firstly proposed in the present invention. Compared with traditional detection methods, the method of the present invention has high sensitivity and good selectivity, and the linear range of detecting the concentration of LPS in an aqueous solution is 10⁻¹⁴-10⁻¹⁰ M.
2) In the present invention, the probe molecule modifying the surface of the gold film has a clear and controllable structure and is easy to be synthesized.
3) In the present invention, the preparation process of SPR chip has simple steps and low cost, which makes the prepared chip have good reproducibility, meet the requirements of batch preparation of industrialized production and be easy to be applied in practical application.

### BRIEF DESCRIPTION OF THE DRAWINGS

Figure 1 is a structural schematic diagram of the sensor chip of the present invention.
Figure 2 is an atomic force microscope (AFM) imaging figure of the gold film plated on the glass substrate by magnetron sputtering technique according to the present invention.
Figure 3 is a selectivity test graph of LPS in an aqueous solution using a sensor chip mounted in a wavelength type surface plasmon resonance sensor apparatus according to the present invention.
Figure 4 is a concentration titration graph of LPS in an aqueous solution using a sensor chip mounted in a wavelength type surface plasmon resonance sensor apparatus according to the present invention, wherein the ordinate represents relative intensity value at resonance wavelength blue shift 12 nm and the abscissa represents the concentration of LPS.
Figure 5 is a graph showing relative intensity of the sensor chip of embodiment 6 versus the concentration of LPS.
Figure 6 is a graph showing relative intensity of the sensor chip of embodiment 15 versus the concentration of LPS.

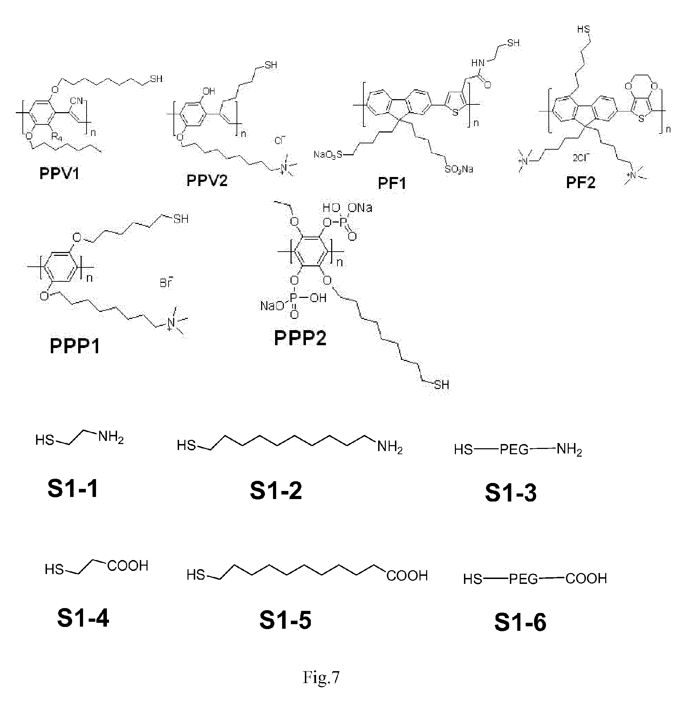

Figure 7 is a structural diagram of a plurality of probe molecules and compound S1 involved in embodiments.

### BRIEF DESCRIPTION OF THE PREFERRED EMBODIMENTS

The present invention will be further illustrated below with reference the embodiments and drawings.

### Embodiment 1

A surface plasmon resonance sensor chip, comprising a glass substrate layer, a gold film layer and a probe molecule layer; the gold film layer is disposed on the glass substrate layer and the probe molecule layer is disposed on the gold film layer; the thickness of the gold film layer is in the range of 10-60 nm; the thickness of the probe molecule layer is in the range of 1-100 nm; the probe molecule layer is the layer formed of one or more probe molecules selected from the group consisting of the following structures: 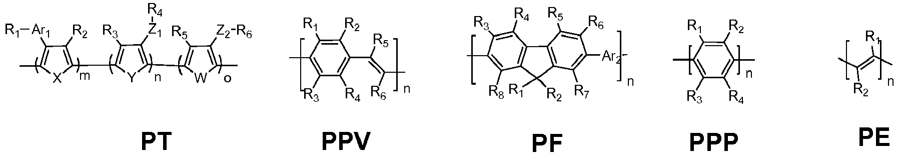 wherein, Ar₁ is thiophene, pyrrole, benzene, naphthalene, anthracene, pyrene, indole, coumarin, fluorescein, carbazole, rhodamine, cyano dyes, fluorene or quinoline.
Ar₂ is one of following structural formulas: 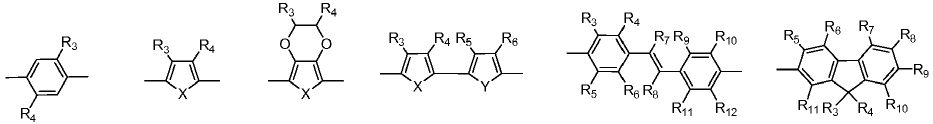 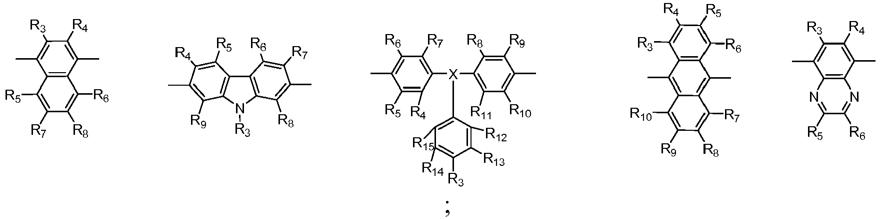 X, Y and W are O, S, N-R₅ or Si-R₆R₇, respectively;
Z₁, Z₂, R₁, R₂, R₃, R₄, R₅, R₆, R₇, R₈, R₉, R₁₀, R₁₁, R₁₂, R₁₃, R₁₄ and R₁₅ are hydrogen atoms, alkyl group, hydroxyl group, mercapto group, carboxyl group, amide, acid anhydride, alkenyl, alkynyl, aryl group, ester group, ether group, amino, cyano group, quaternary ammonium salt, sulphonate, phosphate or polyethylene glycol group, respectively, wherein the number of carbon atoms of the alkyl group, hydroxyl group, mercapto group, carboxyl group, amide, acid anhydride, alkenyl, alkynyl, aryl group, ester group and ether group is in the range of 1-18;
m, n and o are natural numbers in the range of 0-10,000, while m, n and o do not represent 0 simultaneously.

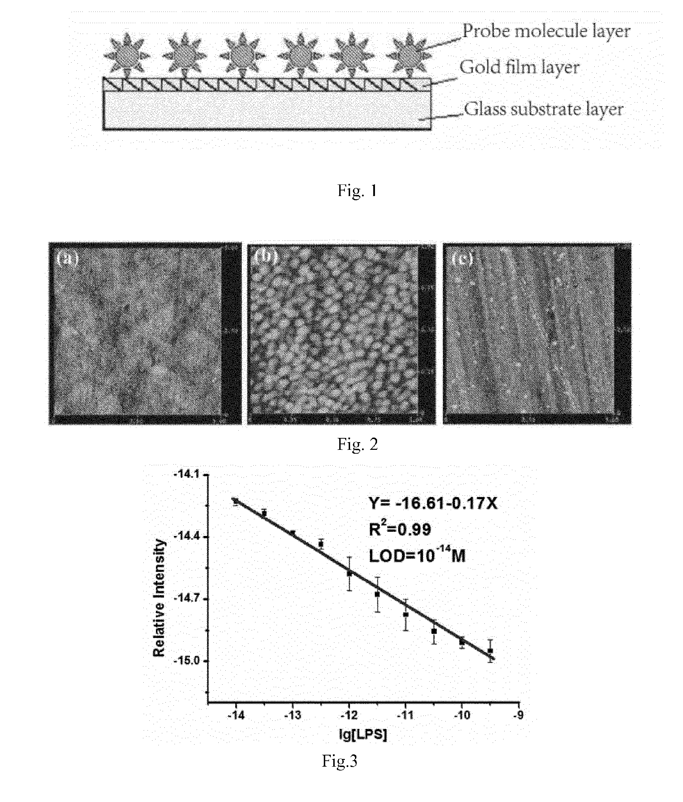

The structure of the surface plasmon resonance sensor chip is shown in Figure 1.

### Embodiment 2

A method for preparing the surface plasmon resonance sensor chip, comprising the steps of:
1) plating a gold (namely Au) film with a thickness of 50 nm on the surface of the glass substrate by vacuum evaporation technology;
2) soaking the glass substrate plated with the gold film obtained from step 1) completely in the probe molecule (PT1) solution with a concentration of 0.01 mg/mL and the solvent of N, N-dimethyl formamide, for 1 hour at room temperature;
3) after soaking completely, taking the glass substrate out and washing it with double distilled water repeatedly to obtain a chip, and then storing the chip in double distilled water for use.

Figure 2 is an atomic force microscope (AFM) imaging figure of the gold film plated on the glass substrate. Figure 2 (a) is the AFM imaging figure of the surface of the gold film in the range of 5 µm; Figure 2(b) is the AFM imaging figure of the surface of the gold film in the range of 1 µm. The white particles of Figure 2 (c) are the probe molecules and the coverage rate of the probe molecules is about 20%.

The surface plasmon resonance sensor chip of embodiment 2 is mounted into an angle modulation type or a wavelength modulation type surface plasmon resonance sensor apparatus. Lipopolysaccharide aqueous solutions with different concentrations are introduced into a flow cell, which can cause the change of the reflection angle. By detecting, it is found that the angle change amount of the reflection angle is linearly proportional to the concentration of the introduced lipopolysaccharide. The result is shown in Figure 3. The shift amount of the surface plasmon resonance peak is linearly proportional to the concentration of the introduced lipopolysaccharide in the range of 10⁻¹⁴-10⁻¹⁰ M.

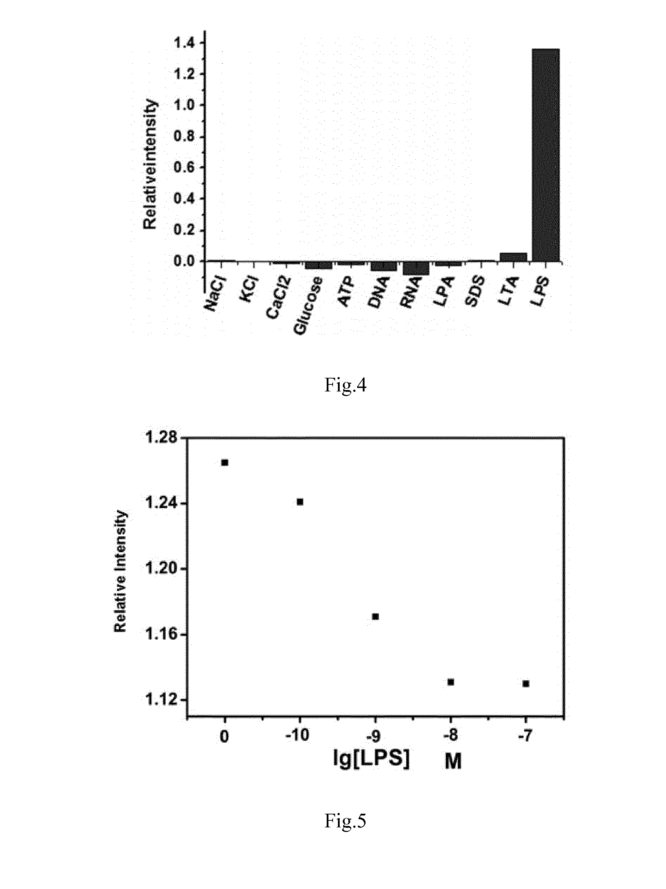

As shown in Figure 4, it will cause a large change of resonance wavelength and relative intensity to mounting this sensor chip into a wavelength modulation type surface plasmon resonance sensor apparatus and introduce lipopolysaccharide aqueous solution with a concentration of 10⁻¹⁰ M into a flow cell. However, it will cause a smaller change of resonance wavelength and relative intensity to introduce other interfering substance aqueous solution with the same concentration, such as sodium chloride, potassium chloride, calcium chloride, glucose, ATP, DNA, RNA, LPA (lysophosphatidic acid), SDS, LTA (lipoteichoic acid), etc. As can be seen, the sensor chip of the present invention has selectivity for lipopolysaccharide.

### Embodiment 3

A method for preparing the surface plasmon resonance sensor chip, comprising the steps of:
1) plating an Au film with a thickness of 50 nm on the surface of the glass substrate by magnetron sputtering technology;
2) soaking the glass substrate plated with gold film of step 1) completely into the probe molecule (PT1) solution with a concentration of 10 mg/mL and the solvent of methyl cyanide, for 10 hours at room temperature;
3) after soaking completely, taking the glass substrate out and washing it with double distilled water repeatedly to obtain a chip, and then storing the chip in double distilled water for use.

### Embodiment 4

A method for preparing the surface plasmon resonance sensor chip, comprising the steps of:
1) plating an Au film with a thickness of 50 nm on the surface of the glass substrate by magnetron sputtering technology;
2) soaking the glass substrate plated with gold film of step 1) completely into the probe molecule (PT1) solution with a concentration of 100 mg/mL and the solvent of 10% tetrahydrofuran and 90% water, for 20 hours at room temperature;
3) after soaking completely, taking the glass substrate out and washing it with double distilled water repeatedly to obtain a chip, and then storing the chip in double distilled water for use.

### Embodiment 5

A method for preparing the surface plasmon resonance sensor chip, comprising the steps of:
1) plating an Au film with a thickness of 50 nm on the surface of the glass substrate by vacuum evaporation technology;
2) soaking the glass substrate plated with gold film of step 1) completely into the probe molecule (PT2) solution with a concentration of 1 mg/mL and the solvent of 10% methyl cyanide and 90% water, for 3 hours at room temperature;
3) after soaking completely, taking the glass substrate out and washing it with double distilled water repeatedly to obtain a chip, and then storing the chip in double distilled water for use.

### Embodiment 6

A method for preparing the surface plasmon resonance sensor chip, comprising the steps of:
1) plating an Au film with a thickness of 50 nm on the surface of the glass substrate by magnetron sputtering technology;
2) soaking the glass substrate plated with gold film of step 1) completely into the probe molecule (PT3) solution with a concentration of 0.01 mg/mL and the solvent of 30% methyl-sulfoxide and 70% water, for 10 hours at room temperature;
3) after soaking completely, taking the glass substrate out and washing it with three times distilled water repeatedly to obtain a chip, and then storing the chip in double distilled water for use.

The surface plasmon resonance sensor chip obtained in embodiment 6 is mounted into a wavelength modulation type surface plasmon resonance sensor apparatus and lipopolysaccharide aqueous solutions with various concentrations are introduced into a flow cell, which can cause a change of resonance wavelength. By detecting, it is found that the change amount of the resonance wavelength is linearly proportional to the concentration of the introduced lipopolysaccharide. The result is shown in Figure 5. The shift amount of the surface plasmon resonance peak is linearly proportional to the concentration of the introduced lipopolysaccharide in the range of 10⁻¹⁰-10⁻⁸ M.

### Embodiment 7

A method for preparing the surface plasmon resonance sensor chip, comprising the steps of:
1) plating an Au film (2) with a thickness of 50 nm on the surface of the glass substrate (1) by vacuum evaporation technology;
2) soaking the glass substrate plated with gold film of step 1) completely into the probe molecule (PT2) solution with a concentration of 1 mg/mL and the solvent of phosphate buffer solution, for 13 hours at room temperature;
3) after soaking completely, taking the glass substrate out and washing it with ultra-pure water repeatedly to obtain a chip, and then storing the chip in double distilled water for use.

### Embodiment 8

A method for preparing the surface plasmon resonance sensor chip, comprising the steps of:
1) plating an Au film with a thickness of 60 nm on the surface of the glass substrate by magnetron sputtering technology;
2) soaking the glass substrate plated with gold film of step 1) completely into the probe molecule (PT4) solution with a concentration of 15 mg/mL and the solvent of physiological saline, for 15 hours at room temperature;
3) after soaking completely, taking the glass substrate out and washing it with double distilled water repeatedly to obtain a chip, and then storing the chip in double distilled water for use.

### Embodiment 9

A method for preparing the surface plasmon resonance sensor chip, comprising the steps of:
1) plating an Au film with a thickness of 60 nm on the surface of the glass substrate by vacuum evaporation technology;
2) soaking the glass substrate plated with gold film of step 1) completely into the probe molecule (PT4) solution with a concentration of 100 mg/mL and the solvent of (2-hydroxyerhyl) piperazine-1-erhaesulfonic acid buffer solution, namely HEPES, for 5 hours at room temperature;
3) after soaking completely, taking the glass substrate out and washing it with double distilled water repeatedly to obtain a chip, and then storing the chip in double distilled water for use.

### Embodiment 10

A method for preparing the surface plasmon resonance sensor chip, comprising the steps of:
1) plating an Au film with a thickness of 60 nm on the surface of the glass substrate by magnetron sputtering technology;
2) soaking the glass substrate plated with gold film of step 1) completely into the probe molecule (PT5) solution with a concentration of 0.01 mg/mL and the solvent of 50% methanol and 50% water, for 18 hours at room temperature;
3) after soaking completely, taking the glass substrate out and washing it with double distilled water repeatedly to obtain a chip, and then storing the chip in double distilled water for use.

### Embodiment 11

A method for preparing the surface plasmon resonance sensor chip, comprising the steps of:
1) plating an Au film with a thickness of 60 nm on the surface of the glass substrate by magnetron sputtering technology;
2a) soaking the glass substrate of step 1) into compound S1-1 solution with a concentration of 0.01 mmol/L, for 1 hour at room temperature; washing it clean with double distilled water repeatedly;
2b) dissolving 10 mg of probe molecules (PT6), 10 mg of NHS and 10 mg of EDC into 50 mL of phosphate buffer solution;
2c) soaking the glass substrate of step 2a) into the mixed solution of step 2b), for 4 hours at room temperature; washing it clean with ethanol and double distilled water repeatedly and carefully, respectively;
3) after soaking completely, taking the glass substrate out and washing it with double distilled water repeatedly to obtain a chip, and then storing the chip in double distilled water for use.

### Embodiment 12

A method for preparing the surface plasmon resonance sensor chip, comprising the steps of:
1) plating an Au film with a thickness of 55 nm on the surface of the glass substrate by magnetron sputtering technology;
2a) soaking the glass substrate of step 1) into compound S1-2 solution with a concentration of 1 mmol/L, for 4 hours at room temperature; washing it clean with double distilled water repeatedly;
2b) dissolving 100 mg of probe molecules (PT6), 50 mg of NHS and 50 mg of EDC into 100 mL of 95% phosphate buffer solution containing 5% methyl cyanide;
2c) soaking the glass substrate of step 2a) into the mixed solution of step 2b), for 20 hours at room temperature; washing it clean with ethanol and double distilled water repeatedly and carefully, respectively;
3) after soaking completely, taking the glass substrate out and washing it with double distilled water repeatedly to obtain a chip, and then storing the chip in double distilled water for use.

### Embodiment 13

A method for preparing the surface plasmon resonance sensor chip, comprising the steps of:
1) plating an Au film with a thickness of 48 nm the surface of the glass substrate by vacuum evaporation technology;
2a) soaking the glass substrate of step 1) into compound S1-3 solution with a concentration of 10 mmol/L, for 10 hours at room temperature; washing it clean with double distilled water repeatedly;
2b) dissolving 50 mg of probe molecules (PT6), 50 mg of NHS and 50 mg of EDC into 100 mL of 95% physiological saline solution containing 5% methyl-sulfoxide;
2c) soaking the glass substrate of step 2a) into the mixed solution of step 2b), for 10 hours at room temperature; washing it clean with ethanol and double distilled water repeatedly and carefully;
3) after soaking completely, taking the glass substrate out and washing it with double distilled water repeatedly to obtain a chip, and then storing the chip in double distilled water for use.

### Embodiment 14

A method for preparing the surface plasmon resonance sensor chip, comprising the steps of:
1) plating an Au film with a thickness of 50 nm on the surface of the glass substrate by vacuum evaporation technology;
2a) soaking the glass substrate of step 1) into compound S1-4 solution with a concentration of 1 mmol/L, for 10 hours at room temperature; washing it clean with double distilled water repeatedly;
2b) dissolving 50 mg of probe molecules (PT7), 50 mg of NHS and 50 mg of EDC into 90 mL of 95% physiological saline solution containing 5% methyl-sulfoxide;
2c) soaking the glass substrate of step 2a) into the mixed solution of step 2b), for 3 hours at room temperature; washing it clean with ethanol and double distilled water repeatedly and carefully, respectively;
3) after soaking completely, taking the glass substrate out and washing it with double distilled water repeatedly to obtain a chip, and then storing the chip in double distilled water for use.

### Embodiment 15

A method for preparing the surface plasmon resonance sensor chip, comprising the steps of:
1) plating an Au film with a thickness of 50 nm on the surface of the glass substrate by magnetron sputtering technology;
2a) soaking the glass substrate of step 1) into compound S1-5 solution with a concentration of 100 mmol/L, for 10 hours at room temperature; washing it clean with double distilled water repeatedly;
2b) dissolving 5 mg of probe molecules (PT7), 10 mg of NHS and 10 mg of EDC into 20 mL of 95% physiological saline solution containing 5% methanol;
2c) soaking the glass substrate of step 2a) into the mixed solution of step 2b), for 3 hours at room temperature; washing it clean with ethanol and double distilled water repeatedly and carefully, respectively.
3) after soaking completely, taking the glass substrate out and washing it with double distilled water repeatedly to obtain a chip, and then storing the chip in double distilled water for use.

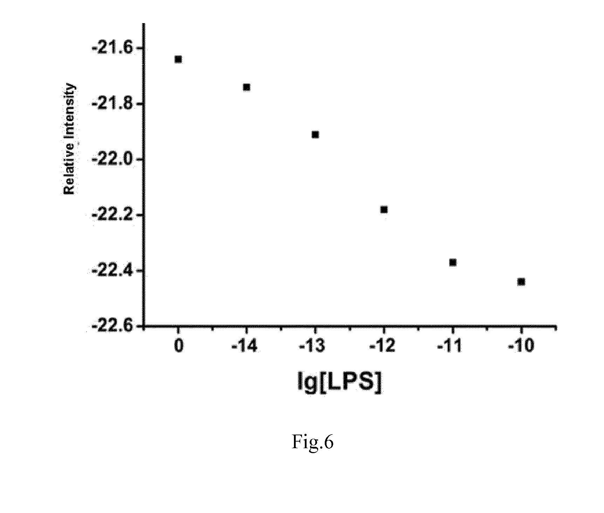

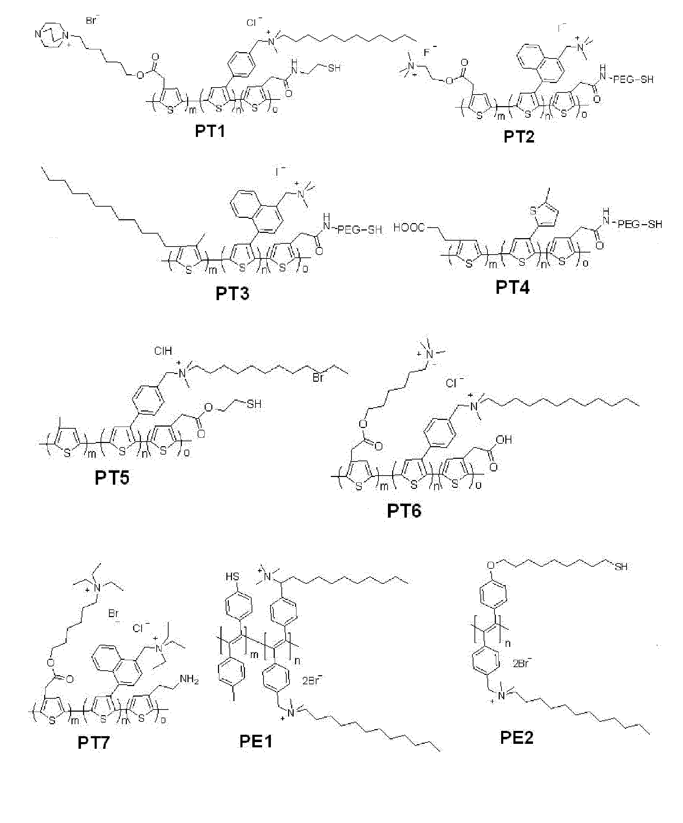

The surface plasmon resonance sensor chip of embodiment 15 is mounted into a wavelength modulation type surface plasmon resonance sensor apparatus and different concentrations of lipopolysaccharide aqueous solutions are introduced into a flow cell, which can cause a change of resonance wavelength. By detecting, it is found that the change amount of the resonance wavelength is linearly proportional to the concentration of the introduced lipopolysaccharide. The result is shown in Figure 6. The shift amount of the surface plasmon resonance peak is linearly proportional to the concentration of the introduced lipopolysaccharide in the range of 10⁻¹⁴-10⁻¹⁰ M.

### Embodiment 16

A method for preparing the surface plasmon resonance sensor chip, comprising the steps of:
1) plating an Au film with a thickness of 60 nm on the surface of the glass substrate by magnetron sputtering technology;
2a) soaking the glass substrate of step 1) into compound S1-6 solution with a concentration of 0.1 mmol/L, for 24 hours at room temperature; washing it clean with double distilled water repeatedly;
2b) dissolving 15 mg of probe molecules (PT7), 20 mg of NHS and 20 mg of EDC into 40 mL of 80% phosphate buffer solution containing 10% methyl cyanide;
2c) soaking the glass substrate of step 2a) into the mixed solution of step 2b), for 13 hours at room temperature; washing it clean with ethanol and double distilled water repeatedly and carefully, respectively;
3) after soaking completely, taking out the glass substrate and washing it with double distilled water repeatedly to obtain a chip, and then storing the chip in double distilled water for use.

### Embodiment 17

A method for preparing the surface plasmon resonance sensor chip, comprising the steps of:
1) plating an Au film with a thickness of 50 nm on the surface of the glass substrate by magnetron sputtering technology;
2) soaking the glass substrate plated with gold film of step 1) completely into the probe molecule (PE1) solution with a concentration of 10 mg/mL and the solvent of methyl cyanide, for 10 hours at room temperature;
3) after soaking completely, taking the glass substrate out and washing it with double distilled water repeatedly to obtain a chip, and then storing the chip in double distilled water for use.

### Embodiment 18

A method for preparing the surface plasmon resonance sensor chip, comprising the steps of:
1) plating an Au film with a thickness of 45 nm on the surface of the glass substrate by magnetron sputtering technology;
2) soaking the glass substrate plated with gold film of step 1) completely into the probe molecule PE2 solution with a concentration of 20 mg/mL and the solvent of methyl-sulfoxide, for 10 hours at room temperature;
3) after soaking completely, taking the glass substrate out and washing it with double distilled water repeatedly to obtain a chip, and then storing the chip in double distilled water for use.

### Embodiment 19

A method for preparing the surface plasmon resonance sensor chip, comprising the steps of:
1) plating an Au film with a thickness of 50 nm on the surface of the glass substrate by magnetron sputtering technology;
2) soaking the glass substrate plated with gold film of step 1) completely into the probe molecule PPV1 solution with a concentration of 10 mg/mL and the solvent of dichloromethane, for 10 hours at room temperature;
3) after soaking completely, taking the glass substrate out and washing it with double distilled water repeatedly to obtain a chip, and then storing the chip in double distilled water for use.

### Embodiment 20

A method for preparing the surface plasmon resonance sensor chip, comprising the steps of:
1) plating an Au film with a thickness of 50 nm on the surface of the glass substrate by magnetron sputtering technology;
2) soaking the glass substrate plated with gold film of step 1) completely into the probe molecule PPV2 solution with a concentration of 10 mg/mL and the solvent of tetrahydrofuran, for 1 hour at room temperature;
3) after soaking completely, taking the glass substrate out and washing it with double distilled water repeatedly to obtain a chip, and then storing the chip in double distilled water for use.

### Embodiment 21

A method for preparing the surface plasmon resonance sensor chip, comprising the steps of:
1) plating an Au film with a thickness of 45 nm on the surface of the glass substrate by magnetron sputtering technology;
2) soaking the glass substrate plated with gold film of step 1) completely into the probe molecule PF solution with a concentration of 10 mg/mL and the solvent of water, for 3 hours at room temperature;
3) after soaking completely, taking the glass substrate out and washing it with double distilled water repeatedly to obtain a chip, and then storing the chip in double distilled water for use.

### Embodiment 22

A method for preparing the surface plasmon resonance sensor chip, comprising the steps of:
1) plating an Au film with a thickness of 50 nm on the surface of the glass substrate by magnetron sputtering technology;
2) soaking the glass substrate plated with gold film of step 1) completely into the probe molecule PF2 solution with a concentration of 10 mg/mL and the solvent of water, for 5 hours at room temperature;
3) after soaking completely, taking the glass substrate out and washing it with double distilled water repeatedly to obtain a chip, and then storing the chip in double distilled water for use.

### Embodiment 23

A method for preparing the surface plasmon resonance sensor chip, comprising the steps of:
1) plating an Au film with a thickness of 48 nm on the surface of the glass substrate by magnetron sputtering technology;
2) soaking the glass substrate plated with gold film of step 1) completely into the probe molecule PPP1 solution with a concentration of 10mg/mL and the solvent of dichloromethane, for 10 hours at room temperature;
3) after soaking completely, taking the glass substrate out and washing it with double distilled water repeatedly to obtain a chip, and then storing the chip in double distilled water for use.

### Embodiment 24

A method for preparing the surface plasmon resonance sensor chip, comprising the steps of:
1) plating an Au film with a thickness of 52 nm on the surface of the glass substrate by magnetron sputtering technology;
2) soaking the glass substrate plated with gold film of step 1) completely into the probe molecule PPP2 solution with a concentration of 10mg/mL and the solvent of water, for 10 hours at room temperature;
3) after soaking completely, taking the glass substrate out and washing it with double distilled water repeatedly to obtain a chip, and then storing the chip in double distilled water for use.

### Embodiment 25

A method for preparing the surface plasmon resonance sensor chip, comprising the steps of:
1) plating an Au film on the surface of the glass substrate, wherein the thickness of the gold film is 10 nm;
2) soaking the glass substrate of step 1) completely into the probe molecule solution with a concentration of 0.01 mg/mL, for 5 minutes;
3) taking the glass substrate out and washing it repeatedly with water to obtain a surface plasmon resonance sensor chip.

The solvent of the probe molecule solution is N, N-dimethyl formamide.

The probe molecule is PPP2.

### Embodiment 26

A method for preparing the surface plasmon resonance sensor chip, comprising the steps of:
1) plating an Au film on the surface of the glass substrate, wherein the thickness of the gold film is 60 nm;
2) soaking the glass substrate of step 1) completely into the probe molecules solution with a concentration of 1000 mg/mL, for 24 hours;
3) taking the glass substrate out and washing it repeatedly with water to obtain a surface plasmon resonance sensor chip.

The solvent of the probe molecule solution is N, N-dimethyl acetamide.

The probe molecule is PF2.

### Embodiment 27

A method for preparing the surface plasmon resonance sensor chip, comprising the steps of:
(1) plating an Au film on the surface of the glass substrate, wherein the thickness of the gold film is 10 nm;
(2) soaking the glass substrate of step (1) into compound S1 solution with a concentration of 0.01 mmol/L, for 1 hour, and then washing the glass substrate clean repeatedly with water for use, mixing the probe molecules with NHS, EDC and a solvent at a mass ratio of 1: 0.1: 0.1: 1 to obtain a mixed solution; soaking the above glass substrate into the mixed solution for 5 minutes, then taking the glass substrate out and washing it with ethanol and water repeatedly;
   wherein, the structural formula of compound S1 is: 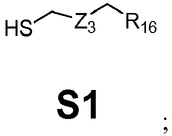 wherein, Z₃ and R₁₆ are hydrogen atoms, respectively.
(3) taking the glass substrate out and washing it repeatedly with water to obtain a surface plasmon resonance sensor chip.

The solvent of compound S1 solution is physiological saline.

The mixed solvent is HEPES buffer solution.

### Embodiment 28

A method for preparing the surface plasmon resonance sensor chip, comprising the steps of:
(1) plating an Au film on the surface of the glass substrate, wherein the thickness of the gold film is 60 nm;
(2) soaking the glass substrate of step (1) into compound S1 solution with a concentration of 1000 mmol/L, for 24 hours, and then washing the glass substrate clean repeatedly with water for use, mixing the probe molecules with NHS, EDC and a solvent at a mass ratio of 1: 100: 100: 1000 to obtain a mixed solution; soaking the above glass substrate into the mixed solution for 24 hours, then taking the glass substrate out and washing it with ethanol and water repeatedly;
   wherein, the structural formula of compound S1 is: 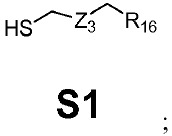 wherein, Z₃ is cyano group and R₁₆ is polyethylene glycol group;
(3) taking the glass substrate out and washing it repeatedly with water to obtain a surface plasmon resonance sensor chip.

The solvent of compound S1 solution is methyl-sulfoxide.

The mixed solvent is N, N-dimethyl acetamide.

### Embodiment 29

The effect of the concentration of probe molecules solution, soaking time, etc. on the coverage rate of the probe molecule on the gold film is measured. To take the probe molecule PT2 as an example and the result is shown in the table below.

| Coverage rate | | Different concentrations | | | | |
|---|---|---|---|---|---|---|
| | | 0.1mg/mL | 1mg/mL | 5mg/mL | 10mg/mL | 100mg/mL |
| Different time | 5 minutes | 5% | 10% | 30% | 35% | 40% |
| | 30 minutes | 7% | 15% | 35% | 45% | 50% |
| | 1 hour | 9% | 25% | 40% | 50% | 60% |
| | 2 hours | 15% | 35% | 45% | 60% | 75% |
| | 5 hours | 20% | 45% | 50% | 70% | 80% |
| | 10 hours | 25% | 50% | 55% | 75% | 85% |
| | 24 hours | 40% | 55% | 65% | 80% | 100% |

The above table shows that with the increase of soaking time, the coverage rate of the probe molecule on the gold film also increases, and with the increase of the concentration of the probe molecule, the coverage rate of the probe molecule on the gold film also increases.

Obviously, the above embodiments of the present invention are only the examples for illustrating clearly the present invention and should not be interpreted as any limitations to the present invention. Various variations or modifications can be made for one skilled in the art based on the above description. Here all of the embodiments can not be provided exhaustively. Obvious variations or modifications derived from the technical solution of the present invention will fall within the protection scope of the present invention.

## Claims

1. A surface plasmon resonance sensor chip, comprising:
a glass substrate layer, a gold film layer, and a probe molecule layer;
wherein the gold film layer is disposed on the glass substrate layer and the probe molecule layer is disposed on the gold film layer;
wherein the probe molecule layer is the layer formed of one or more probe molecules selected from the group consisting of the following structures: 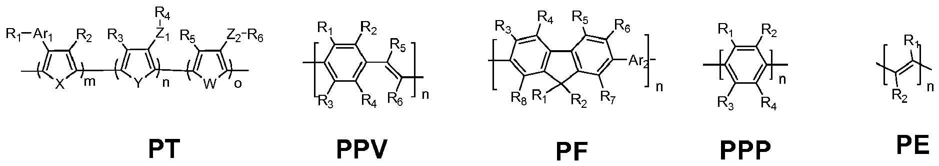
wherein, Ar₁ is thiophene, pyrrole, benzene, naphthalene, anthracene, pyrene, indole, coumarin, fluorescein, carbazole, rhodamine, cyano dyes, fluorene or quinoline;
Ar₂ is one of the following structural formulas: 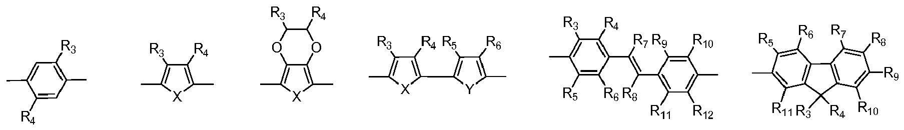 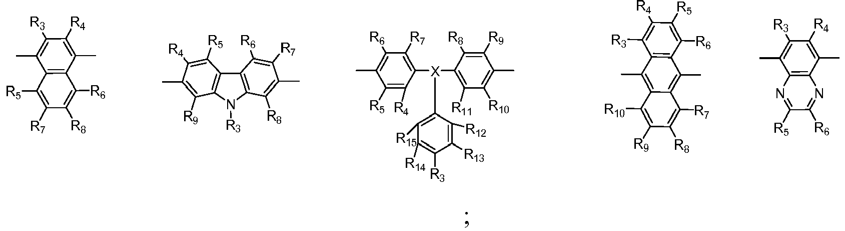
X, Y and W are O, S, N-R₅ or Si-R₆R₇, respectively;
Z₁, Z₂, R₁, R₂, R₃, R₄, R₅, R₆, R₇, R₈, R₉, R₁₀, R₁₁, R₁₂, R₁₃, R₁₄ and R₁₅ are hydrogen atoms, alkyl group, hydroxyl group, mercapto group, carboxyl group, amide, acid anhydride, alkenyl, alkynyl, aryl group, ester group, ether group, amino, cyano group, quaternary ammonium salt, sulphonate, phosphate or polyethylene glycol group, respectively, wherein the number of carbon atoms of the alkyl group, hydroxyl group, mercapto group, carboxyl group, amide, acid anhydride, alkenyl, alkynyl, aryl group, ester group and ether group is in the range of 1-18;
m, n and o are natural numbers in the range of 0-10,000, while m, n and o do not represent 0 simultaneously.

2. The surface plasmon resonance sensor chip of claim 1, wherein the thickness of the gold film layer is in the range of 10-60 nm; the thickness of the probe molecule layer is in the range of 1-100 nm; the coverage rate of the probe molecule layer on the gold film layer is in the range of 5%-100%.

3. A method for preparing the surface plasmon resonance sensor chip of claim 1 or 2, comprising the following steps:
1) plating Au on the surface of the glass substrate;
2) soaking the glass substrate obtained from step 1) completely in a probe molecule solution with a concentration of 0.01-1000 mg/mL, for 5 minutes-24 hours;
3) taking the glass substrate out of the probe molecule solution and washing it repeatedly with water to obtain the surface plasmon resonance sensor chip.

4. The method for preparing the surface plasmon resonance sensor chip of claim 3, wherein the thickness of the gold film is in the range of 10-60 nm.

5. The method for preparing the surface plasmon resonance sensor chip of claim 3, wherein the solvent of the probe molecule solution is selected from:
physiological saline, (2-hydroxyerhyl) piperazine-1-erhaesulfonic acid (HEPES) buffer solution or phosphate buffer solution; or
one solvent selected from the group consisting of methanol, ethanol, methyl cyanide, dichloromethane, chloroform, tetrahydrofuran, methyl-sulfoxide, N,N-dimethyl formamide, and N,N-dimethyl acetamide or a mixed solvent thereof; or
a mixture of the one solvent or the mixed solvent and water in any proportions.

6. A method for preparing the surface plasmon resonance sensor chip of claim 1 or 2, comprising the following steps:
1) plating Au on the surface of the glass substrate;
2) soaking the glass substrate obtained from step (1) into compound S1 solution with a concentration of 0.01-1000 mmol/L, for 1-24 hours; then washing the glass substrate repeatedly with water for use; mixing the probe molecules with NHS, EDC and a solvent at a mass ratio of 1: 0.1-100: 0.1 -100: 1-1000 to obtain a mixed solution; soaking the glass substrate into the mixed solution for 5 minutes-24 hours, then taking the glass substrate out of the mixed solution and washing it with ethanol and water repeatedly;
wherein, the structural formula of compound S1 is: 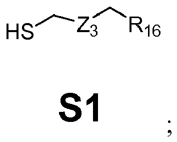 wherein, Z₃ and R₁₆ are hydrogen atoms, alkyl group, hydroxyl group, mercapto group, carboxyl group, amide, acid anhydride, alkenyl, alkynyl, aryl group, ester group and ether group, amino, cyano group or polyethylene glycol group, respectively, wherein the number of carbon atoms of the alkyl group, hydroxyl group, mercapto group, carboxyl group, amide, acid anhydride, alkenyl, alkynyl, aryl group, ester group and ether group is in the range of 1-18;
3) washing the glass substrate repeatedly with water to obtain the surface plasmon resonance sensor chip.

7. The method for preparing the surface plasmon resonance sensor chip of claim 6, wherein the thickness of the gold film is in the range of 10-60 nm.

8. The method for preparing the surface plasmon resonance sensor chip of claim 6, wherein the compound S1 is dissolved in physiological saline, (2-hydroxyerhyl) piperazine-1-erhaesulfonic acid (HEPES) buffer solution or phosphate buffer solution; or one solvent selected from the group consisting of methanol, ethanol, methyl cyanide, dichloromethane, chloroform, tetrahydrofuran, methyl-sulfoxide, N,N-dimethyl formamide and N,N-dimethyl acetamide or a mixed solvent thereof; or a mixture of the one solvent or the mixed solvent and water in any proportions, to form the compound S1 solution;
wherein the solvent is selected from:
physiological saline, HEPES buffer solution or phosphate buffer solution; or
one solvent selected from the group consisting of methanol, ethanol, methyl cyanide, dichloromethane, chloroform, tetrahydrofuran, methyl-sulfoxide, N,N-dimethyl formamide and N,N-dimethyl acetamide or a mixed solvent thereof; or
a mixture of the one solvent or the mixed solvent and water in any proportions.

9. A use of the surface plasmon resonance sensor chip of any one of claims 1-8 for detecting lipopolysaccharide in an aqueous solution.

10. The use of the surface plasmon resonance sensor chip of claim 9, wherein the sensor chip is mounted into an angle modulation type or a wavelength modulation type surface plasmon resonance sensor apparatus, lipopolysaccharide aqueous solutions with various concentrations are introduced into a flow cell and the lipopolysaccharide is detected by detecting a surface plasmon resonance peak shift.
